(19) Europäisches Patentamt / European Patent Office / Office européen des brevets

(11) **EP 3 610 858 B1**

(12) **EUROPEAN PATENT SPECIFICATION**

(45) Date of publication and mention of the grant of the patent:
**25.03.2026 Bulletin 2026/13**

(21) Application number: **18784969.0**

(22) Date of filing: **12.04.2018**

(51) International Patent Classification (IPC):
*A61K 9/10* (2006.01)    *A61K 47/36* (2006.01)
*A61K 47/10* (2017.01)    *A61K 33/06* (2006.01)
*A61P 1/12* (2006.01)

(52) Cooperative Patent Classification (CPC):
**A61P 1/12; A61K 9/10; A61K 33/06; A61K 47/10; A61K 47/36;** Y02A 50/30

(86) International application number:
**PCT/KR2018/004304**

(87) International publication number:
**WO 2018/190658 (18.10.2018 Gazette 2018/42)**

(54) **SMECTITE SUSPENSION LIQUID COMPOSITION AND METHOD FOR PREPARING SAME**

SMEKTITTON-SUSPENSIONSFLÜSSIGKEITSZUSAMMENSETZUNG UND HERSTELLUNGSVERFAHREN DAFÜR

COMPOSITION LIQUIDE DE SUSPENSION DE SMECTITE ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.04.2017 CN 201710240595**

(43) Date of publication of application:
**19.02.2020 Bulletin 2020/08**

(73) Proprietors:
• **Daewoong Pharmaceutical Co., Ltd.**
  **Hwaseong-si, Gyenoggi-do 18623 (KR)**
• **Liaoning Daewoong Pharmaceutical Co., Ltd.**
  **Benxi, Liaoning 117004 (CN)**

(72) Inventors:
• **CHANG, Ye**
  **Benxi**
  **Liaoning 117004 (CN)**
• **LI, Qing Ri**
  **Benxi**
  **Liaoning 117004 (CN)**
• **SEOL, Sang Ho**
  **Benxi**
  **Liaoning 117004 (CN)**
• **LI, Tie**
  **Benxi**
  **Liaoning 117004 (CN)**
• **TONG, Chao**
  **Benxi**
  **Liaoning 117004 (CN)**

(74) Representative: **Eisenführ Speiser**
  **Patentanwälte Rechtsanwälte PartGmbB**
  **Postfach 10 60 78**
  **28060 Bremen (DE)**

(56) References cited:
EP-A1- 2 386 289        WO-A2-2005/041876
WO-A2-2010/102923       CN-A- 101 065 111
CN-A- 101 129 326       CN-A- 102 218 078
CN-A- 106 265 724       CN-A- 106 281 044
US-A- 4 942 042         US-A- 5 272 137
US-A1- 2015 306 113     US-B2- 7 135 197

**Description**

[Technical Field]

The present invention relates to pharmaceutical formulations, and more particularly, to a suspension composition comprising smectite and a method of preparing the same.

[Background Art]

Diarrhea can be classified into, according to the causes thereof, osmotic diarrhea due to substances that cannot be absorbed into the intestinal tract, secretory diarrhea caused by secretion accelerators such as bacterial toxins, bile acids, fatty acids, laxatives, and the like without structural damage to the intestinal mucosa, and mucosal damage-related diarrhea due to structural damage to the intestinal mucosa, such as inflammatory bowel disease, ischemic bowel disease, and the like, and can be classified into, according to the symptoms thereof, chronic diarrhea and acute diarrhea.

Smectite is a natural clay composed of aluminum hydroxide-magnesium carbonate sorosilicate, has a layered structure and a heterogeneous charge distribution, is capable of immobilizing and inhibiting viruses, pathogenic bacteria, and toxins produced thereby present in the digestive tract, and is able to cover the digestive mucous membrane. In addition, smectite has an effect of treating diarrhea by qualitatively and quantitatively restoring the protective function of the mucosal barrier against attack factors through mutual binding to mucous glycoproteins.

Smectite powder (Smecta®) was released in 1975 for the treatment of diarrhea in France, and in 2002 it has already been successfully used for the treatment of diarrhea in more than 60 countries around the world. Smectite powder was launched in China in 1990, and large-scale clinical studies have been conducted. The research results show that smectite powder is highly effective for the treatment of diseases such as acute/chronic diarrhea, peptic ulcer, colitis, reflux esophagitis, and the like in adults and children.

Currently, smectite has a wide range of clinical applications of diarrhea treatment in China, and as formulations released, there are powders, granules, dispersible tablets, and suspensions.

Thereamong, powders should be dispersed by adding water thereto before administration and there is a problem in that, depending on the user, the dispersion may not be uniform or powder may be agglomerated to form an aggregate. In contrast, suspension preparations can be taken in a uniform form compared to a dispersing agent, and have the effects of not only increasing user's drug compliance but also exhibiting faster efficacy.

However, in conventional suspensions, to satisfy conditions such as a sinking rate and the like, a method of reducing particle size through grinding after raw materials are mixed has been used in order to slow down the rate at which the raw materials are precipitated in liquid. However, the grinding process requires a large grinding apparatus in a mass production facility, is complicated, and may still cause precipitation problems during long-term storage of a prepared suspension. In addition, when a suspension comprises a clay component such as smectite, the viscosity of the suspension may increase, and in the preparation of a suspension, uniformity, a sinking rate, and the like may be worsened, and it is difficult to meet user's preference.

Document EP 2 386 289 A1 discusses clay compositions which are useful for the treatment of acute and chronic diarrhoea.

Document CN102218078A deals with a rifaximin suspension that contains Montmorillonitum and a preparation method thereof.

Document WO 2005/041876 A3 concerns spill resistant formulations comprising either a weak base or a weak acid as the pharmaceutical ingredient, a liquid base, a clay and a water soluble cellulose ether.

Document WO 2010/102923 A3 reports of aqueous riluzole suspensions.

In document CN 106281044 A is discussed a preparation method of a floating type aqueous polishing liquid.

Document US 4,942,042 discloses anti-diarrhea compositions.

Document CN 106265724 A deals with a smectite turbid liquor.

Document CN 101129326 A concerns a method for preparing an ascanite suspension.

In document CN 101065111 A, a liquid pharmaceutical composition is described which contains at least one basic water-insoluble active agent.

Document US 2015/306113 A1 describes bismuth containing liquid pharmaceutical suspensions.

Document US 7,135,197 B2 pertains to a stable pharmaceutical composition useful for treating gastrointestinal disorders.

Document US 5,272,137 deals with an aqueous pharmaceutical suspension for pharmaceutical actives.

Therefore, there is still a need to develop a suspension composition that satisfies excellent sinking rate conditions as a suspension, meets user preference, and is easy to prepare.

[Disclosure]

[Technical Problem]

Therefore, the present invention has been made in view of solving the above problems, and it is one object of the present invention to provide a smectite suspension which does not undergo layer separation even after long-term storage and has high user preference, and exhibits excellent formulation uniformity in the preparation process thereof, and a method of preparing the smectite suspension.

As a result of having made efforts to develop a smectite suspension agent which satisfies all of ease of preparation, formulation uniformity, and a high sinking rate, and has high user preference, the inventors of the present invention confirmed that, when a suspension comprises both a polymeric suspending agent and a low molecular suspending agent, the suspension has excellent formulation characteristics, and thus completed the present invention. In particular, when the suspension of the present invention comprises certain amounts of a polymeric suspending agent and a low molecular suspending agent, the suspension satisfies all of ease of preparation, formulation uniformity, and high sinking rate, and also has high user preference.

[Technical Solution]

The present invention provides a smectite suspension comprising smectite; and a polymeric suspending agent and a low molecular suspending agent. The present invention is characterized in that, unlike conventional suspensions comprising only a suspending agent, the smectite suspension is prepared using a mixture of a polymeric suspending agent and a low molecular suspending agent.

The present invention also provides a method of preparing a smectite suspension, the method comprising preparing a mixed suspending agent by mixing a swollen polymeric suspending agent and a low molecular suspending agent and heating the resulting mixture at 90 °C to 95 °C for 25 minutes to 45 minutes; and preparing a smectite suspension by mixing the mixed suspending agent with an aqueous smectite dispersion and heating the resulting mixture at 85 °C to 95 °C for 15 minutes to 45 minutes.

[Advantageous effects]

As apparent from the fore-going description, a smectite suspension of the present invention comprises both a polymeric suspending agent and a low molecular suspending agent, and thus can enhance a suspending effect of a product, can provide excellent texture and patient compliance via a decrease in viscosity thereof, and can be prepared through a mechanical stirring process, thus reducing manufacturing costs. In addition, the smectite suspension does not cause layer separation even after long-term storage thereof and exhibits a very high sinking rate.

[Description of Drawings]

The above and other objects, features and other advantages of the present invention will be more clearly understood from the following detailed description taken in conjunction with the accompanying drawings, in which:

FIG. 1 is an image showing the results of confirming a state of a smectite suspension prepared according to Example 1 after being maintained for 3 hours; and

FIG. 2 is an image showing the results of confirming a state of a smectite suspension composition having a configuration different from the present invention and sold in China after being maintained for 3 hours.

[Mode]

The present invention provides a smectite suspension comprising smectite; and a polymeric suspending agent and a low molecular suspending agent.

Smectite is a kind of natural clay containing large amounts of various minerals such as aluminum, magnesium, and the like, and has been used as an adsorbent anti-diarrhea agent for a long period of time. Due to the structure of smectite, it has a space that can contain water, and thus absorbs moisture and thereby stops diarrhea, and absorbs and excretes pathogenic bacteria, toxins, and viruses that can cause diarrhea in the intestines. Smectite also acts to protect the gastrointestinal mucosa and relieve pain by covering the damaged mucosa. However, since smectite is a clay component, it is difficult to prepare a suspension by dispersing smectite in an aqueous solution due to increased viscosity of the suspension. In particular, in the case of preparing a suspension having a high viscosity, it is difficult to agitate and the introduction of a new facility is required, and therefore, there is also a problem of increasing manufacturing costs.

The present invention recognizse above problems, and is characterized in that the present invention comprises both a polymeric suspending agent and a low molecular suspending agent to improve formulation characteristics of the smectite suspension to achieve ease of preparation. In this case, it may be possible to provide a smectite suspension which has viscosity suitable for use as a pharmaceutical agent and exhibits excellent ease of preparation, dispersibility, and storage stability (a sinking rate of 0.98 or higher).

The smectite comprises dioctahedral species, such as montmorillonite and beidellite, and trihedrons, such as hectorite and saponite, and in the present invention, specific experiments were carried out on a suspending fomulation containing montmorillonite.

The smectite may be comprised in an amount of 8 %(w/v) to 15 %(w/v) with respect to the total amount of the suspension. When the amount of the smectite is greater than the above range, the suspension may exhibit poor formulation properties, and when the amount of the smectite is less than the above range, the effect obtained by smectite may be insignificant.

As used herein, the term "suspending agent" refers to a component that aids in the dispersion of smectite and other components in a solution. Thereamong, the polymeric suspending agent means a substance having a higher molecular weight than the low molecular suspending agent.

The polymeric suspending agent is comprised in an amount of greater than 0.05 %(w/v) to less than 0.45 %(w/v) with respect to the total amount of the smectite suspension. When the amount of the polymeric suspending agent is equal to or less than the above range, the suspension exhibits a poor sinking rate, and when the amount of the polymeric suspending agent is equal to or greater than the above range, it is difficult to prepare the suspension due to difficulty in agitation. In particular, when the amount of the polymeric suspending agent is equal to or greater than the above range, it is difficult to perform uniform agitation, and thus the dispersibility of the suspension decreases, and accordingly, the quantity of defective products having an insufficient content increases in the production of products.

The polymeric suspending agent may be one or more selected from the group consisting of microcrystalline cellulose, carboxymethylcellulose, hydroxypropyl cellulose, xanthan gum, carrageenan, and carbomer. The cellulose may comprise commercially available celluloses. As an example, RC-591(Avicel®) may be comprised in the cellulose-type polymer suspending agent, but the present invention is not limited thereto. The polymeric suspending agent is preferably xanthan gum.

The low molecular suspending agent is comprised in an amount of 8.5 %(w/v) to 14 %(w/v) with respect to the total amount of the smectite suspension. When the amount of the low molecular suspending agent is less than the above range, the suspension may exhibit a poor sinking rate, and when the amount of the low molecular suspending agent is greater than the above range, texture and preference may worsen. In particular, it may be more difficult for a user to take the suspension.

The low molecular suspending agent may be glycerin and/or sorbitol, preferably glycerin.

The smectite suspension of the present invention may comprise xanthan gum as the polymeric suspending agent and glycerin as the low molecular suspending agent. In this case, thanks to the two suspending agents, the suspension may exhibit a high sinking rate, and an appropriate viscosity for the suspension may be imparted.

A total amount of the polymeric suspending agent and the low molecular suspending agent in the smectite suspension of the present invention may range from 10 %(w/v) to 15 %(w/v) with respect to the total suspension composition. When the above range is satisfied, the suspension exhibits a high sinking rate and satisfies a viscosity range of 10 mPa·s to 130 mPa·s, thus exhibiting high product production efficiency (low defect rate) and excellent texture.

Preferably, when the smectite suspension of the present invention comprises xanthan gum as the polymeric suspending agent and glycerin as the low molecular suspending agent, a total amount of xanthan gum and glycerin may range from 10 %(w/v) to 15 %(w/v) with respect to the total suspension composition. In particular, in this case, the suspension of the present invention may comprise xanthan gum and glycerin in a weight ratio of 1:26 to 200. When the above range is satisfied, it may be possible to provide a suspension which exhibits a very high sinking rate and has a viscosity of 10 mPa·s to 130 mPa·s, thus satisfying high product preparation efficiency (low defect rate) and excellent texture. Since there are difficulties in increasing the ease of preparation and storage stability of medicines, the present invention has an advanced effect in that the ease of preparation and storage stability of the smectite suspension is enhanced.

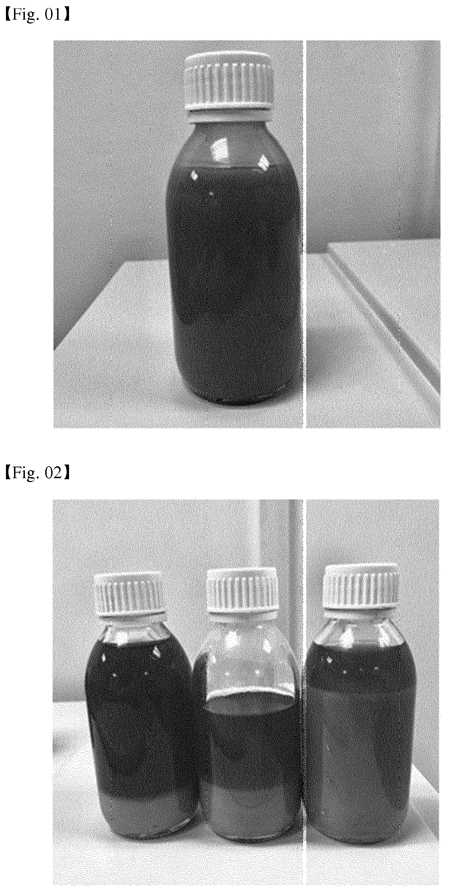

The smectite suspension of the present invention may comprise 8 %(w/v) to 12 %(w/v) of smectite, 0.1 %(w/v) to 0.4 %(w/v) of xanthan gum as the polymeric suspending agent, and 8.5 %(w/v) to 14 %(w/v) of glycerin as the low molecular suspending agent. A suspension that satisfies the above range has a very high sinking rate, which approximates to 1, and does not cause layer separation even after storage for 3 hours. In addition, excellent texture and high preference were experimentally confirmed (see FIG. 1).

The suspension may further comprise 5 %(w/v) to 9 %(w/v) of sucrose, 0.05 %(w/v) to 0.09 %(w/v) of sodium benzoate, 0.09 %(w/v) to 0.35 %(w/v) of citric acid, and 0.055 %(w/v) to 0.095 %(w/v) of sodium citrate. When the above conditions are satisfied, a smectite suspension with a very high sinking rate and excellent texture may be provided.

In particular, as illustrated in FIGS. 1 and 2, as a result of examining the states of a smectite suspension prepared according to Example 1 of the present invention and a smectite suspension preparation currently sold in China, after being left for 3 hours, it was confirmed that the smectite suspension of the present invention (a sinking rate of 0.99) exhibited excellent stability and did not show layer separation (see FIG. 1), whereas the commercially available suspension preparation (a sinking rate of 0.70) clearly showed layer separation after 3 hours (see FIG. 2). Thus, it can be seen that the suspension preparation of the present invention has excellent formulation stability compared to the commercially available conventional suspension preparation.

The suspension of the present invention may have a pH of 4.0 to 9.0, preferably 4.5 to 8.5.

The suspension of the present invention may further comprise one or more components selected from the group consisting of a sweetener, a preservative, a flavor, a pH adjusting agent, and a colorant.

The term "sweetener" as used herein refers to a food additive that exhibits (reproduces) sweetness but generally has less calories, and comprises both natural and synthetic sweeteners. The sweetener may be applied to the present invention without limitation, as long as it can be commonly used in the food and pharmaceutical fields.

Examples of the sweetener comprise xylose, ribose, glucose (dextrose), mannose, galactose, fructose (levulose), sucrose (sugar), maltose, hydrogenated maltose (maltitol), invert sugar (a mixture of glucose and fructose derived from sucrose), partially hydrolyzed starch, corn syrup solids, monosaccharides, disaccharides, and polysaccharides such as dihydrochalcones, monellin, stevioside, and glycyrrhizin. Suitable water soluble artificial sweeteners comprise water soluble saccharin salts (i.e., sodium or calcium saccharin salts), cyclamate salts, sodium, ammonium or calcium salts of 3,4-dihydro-6-methyl-1,2,3-oxathiazin-4-one-2,2-dioxide, potassium salts of 3,4-dihydro-6-methyl-1,2,3-oxathiazin-4-one-2,2-dioxide (acesulfame-K) and saccharin in a free acid form. Water-soluble sweeteners are derived from natural water-soluble sweeteners, for example chlorinated derivatives of general sugar (sucrose) known in the product manual for sucralose. Suitable sweeteners may comprise natural sweeteners from plants, such as a Thaumatin or Stevia extract.

As used herein, the term "preservative" means a pharmaceutically acceptable substance that prevents microbial growth or degradation by unwanted chemical changes. The preservatives have bactericidal and/or fungicidal or antioxidant properties, and those commonly used in the art may be used in the present invention without limitation.

The preservative comprises compounds having bactericidal and/or fungicidal or antioxidant properties, such as citric acid, ascorbic acid, lauryl alcohol, sorbic acid, sodium benzoate, sodium methyl paraben, sodium propyl paraben, parahydroxymethylbenzoate, parahydroxypropylbenzoate, parahydroxybenzoic acid, potassium sorbate, propylene glycol, chlorhexidine gluconate, bronopol, cetylpyridinium chloride, glycerin (glycerol), alpha tocopherol, and butylated hydroxy toluene (BHT), but the present invention is not limited thereto.

As used herein, the term "flavor" or "aroma" comprises flavor ingredients or compositions commonly used in the food industry, whether of natural or synthetic origin. The flavor or aroma comprises a single compound or a mixture. Specific examples of such compounds can be found, for example, in literature [Fenaroli's Handbook of Flavor Ingredients, 1975, CRC Pres] or as commercially available products.

As used herein, the term "pH adjusting agent" refers to an excipient used to adjust the pH of the suspension to a desired value, and may be, for example, without being limited to, citric acid, sodium citrate, or ascorbic acid, and those commonly used in the art to which the present invention pertains may be used in the present invention without limitation.

As used herein, the term "colorant" is intended to be comprised to change the color of the suspension, and may be used to prepare a suspension having a desired color in the formulation. Those commonly used in the art to which the present invention pertains may be comprised in the present invention with limitation. For example, erythrosine may be comprised in the smectite suspension.

As used herein, the term "viscosity" is a measure of the resistance of a fluid to flow. As the viscosity unit, P (poise), cP (centipoise), or Pa·s (pascal second), or mPa·s (milli pascal second) is used. The method used for measuring the viscosity according to the present invention may be used without limitation as long as it is commonly used in the art to which the present invention pertains. For example, a rotational viscometer is used. A rotational viscometer measures viscosity by measuring the operating torque of a cylindrical rotor immersed in a sample. Specifically, the viscosity may be measured by measuring the operating torque of a motorized cylindrical rotor inserted in the sample and rotating at a constant speed. The rotational viscometer performs a measurement method assuming that the viscosity is directly proportional to the operating torque required to produce a constant rotational movement.

When the viscosity of the suspension of the present invention is measured in 20 stabilized sections within 60 seconds under a 25 °C temperature condition at a rate of 100 s$^{-1}$ to 1400 s$^{-1}$ using a RheolabQC viscometer, the viscosity ranges from 10 mPa·s to 130 mPa·s, and when the above conditions were satisfied, a suspension with excellent texture and excellent ease of administration may be provided. In addition, the suspension of the present invention has excellent dispersibility, and thus may reduce a defect rate in preparation thereof and exhibit a high sinking rate.

As used herein, the term "sinking rate" refers to the rate at which and extent to which particles sink in a fluid, and in the case of suspension, a sinking rate of a certain level or higher (when 1 is a maximum level, 0.9 or higher is required) should be satisfied to get permission to sell the suspension as a drug. In this regard, the suspension of the present invention which comprises both the polymeric suspending agent and the low molecular suspending agent has a very high sinking rate.

The present invention also provides a method of preparing a smectite suspension, the method comprising: preparing a mixed suspending agent by mixing a polymeric suspending agent and a low molecular suspending agent and heating the resulting mixture at 90 °C to 95 °C for 25 minutes to 45 minutes; and preparing a smectite suspension by mixing the mixed suspending agent with an aqueous smectite dispersion and heating the resulting mixture at 85 °C to 95 °C for 15 minutes to 45 minutes.

The preparation of the mixed suspending agent may be performed by completely dispersing the polymeric suspending agent, and then completely dispersing the added low molecular weight suspending agent. The dispersion of the polymeric suspending agent may be performed by agitation for 35 minutes to 45 minutes, and the dispersion of the low molecular suspending agent may be performed while stirring for 3 minutes to 7 minutes.

The preparation method may further comprise adding a pH adjusting agent while stirring. When two or more kinds of pH adjusting agents are used, the addition of the pH adjusting agent may be performed by adding and dispersing one kind of pH adjusting agent, and then adding and dispersing another kind of pH adjusting agent.

The preparation method may further comprise performing a cooling process after the mixing of the mixed suspending agent with the aqueous smectite dispersion. The resulting mixture may be cooled to room temperature. The cooling method may be all cooling methods commonly used in the art, for example, room-temperature cooling.

The preparation method may further comprise adding, to the cooled mixture, any one ingredient selected from the group consisting of sweeteners, preservatives, flavors, and colorants and mixing the same. When two or more of the above ingredients are comprised, the above process may be performed by dispersing one ingredient, and then adding another ingredient. Preferably, the addition process may proceed while stirring is continued.

The preparation method of the present invention is characterized in that the polymeric suspending agent and the low molecular suspending agent are first mixed to prepare a mixed suspending agent, and then the mixed suspending agent is mixed with an aqueous smectite dispersion. It was confirmed that, when a suspension preparation containing smectite is prepared using the method according to the present invention, an excessive increase in viscosity of the composition may be prevented, and the composition exhibited excellent dispersibility, thus providing a suspension with a high sinking rate. The sinking rate of the suspension preparation is a very important requirement in terms of the approval as a drug, and a poor sinking rate may cause storage problems of the drug, and drug uniformity deteriorates, thus reducing treatment effects for patients who take the drug. In this regard, the present invention has technical significance in that a suspension with a high sinking rate may be provided.

A stirrer used in the present invention for the preparation of the suspension may be any stirrer commonly used in the art to which the present invention pertains without limitation, particularly a blade-paddle, but the present invention is not limited thereto.

In the preparation of the suspension, the stirring rate may range from 100 rpm to 1,000 rpm, preferably 400 rpm to 600 rpm, but the present invention is not limited thereto.

Hereinafter, the present invention will be described in detail with reference to the following Examples and Experimental Examples.

## [Examples]

### [Experimental Example 1] Preparation of Suspension and Confirmation of Viscosity Properties of Formulation

A suspension comprising a clay component such as smectite may have a high viscosity. However, when the viscosity of the suspension is too high, there may be a problem in that the defect rate of a product increases during preparation thereof, it may be inconvenient for a user to take a drug, and drug efficacy may be reduced. Therefore, experiments were conducted to confirm the viscosity properties of a smectite suspension and an effect on formulation properties, especially viscosity of the suspension. The specific composition of a suspension of the present invention is the same as shown in Table 2 (Example 1), and suspensions having the compositions of Comparative Examples 1 to 3, which are known as the compositions of smectite suspensions in the art to which the present invention pertains were separately prepared to measure the viscosity of each suspension.

Specifically, for pH adjustment, ascorbic acid (2.00 g) and citric acid (4.00 g) were dissolved in 200 ml of purified water at room temperature, and potassium sorbate was separately dissolved in 200 ml of purified water at room temperature. In addition, 500 g of polymer maltitol liquid (polymer suspending agent, maltitol syrup polysorb 75/55A or maltitol syrup lycasin 85/55), 10 g of aromatic caramel (flavor), and 1.2 g of sodium saccharin (sweetener) were added to a separate container while stirring, and then the ascorbic acid/citric acid solution was added thereto while stirring was continued, and then moved to a reactor. While an appropriate amount of diosmectite, which had been filtered through a 1 mm sieve before use, was added in aliquots to a blade-paddle homogenizer reactor, the stirring was continued to homogenize the mixture. A preservative solution was added to the reactor and stirred to homogenize the mixture. After 15 minutes of stirring, about 2 kg of a pale creamy colored suspension of Comparative Example 1 was obtained. During preparation thereof, the internal temperature of the suspension was maintained at 25 °C, and the temperature of the reactor was adjusted through a cooling thermostat device, and the stirring rate of the reactor was 1,200 rpm. Stirring was performed for 15 minutes, and a blade-paddle-type stirrer was used.

In the case of Example 1, 300 g of white sugar was completely dissolved in 300 ml of purified water, 3.5 g of xanthan gum was added together with 100 ml of purified water, and stirred for 40 minutes to completely disperse the components. Subsequently, 115 g of glycerin was added and stirred for 5 minutes, 1.25 g of citric acid was added and stirred for 5 minutes, 0.75 g of sodium citrate was added and stirred for 5 minutes, 100 g of diosmectite was added together with 300 ml of purified water, the temperature was raised to 90 °C, and then the resulting solution was stirred for 30 minutes to 40 minutes and cooled to room temperature. After confirming the cooling to room temperature, 0.7 g of sodium benzoate was added and stirred for 5 minutes, 6 g of vanilla flavor was added and stirred for 5 minutes, 6 g of strawberry essence was added and stirred for 5 minutes, and 0.045 g of erythrosine was added, followed by quantification to 1 L with purified water and stirring for 10 minutes. The stirring rate of the reactor was 500 rpm, and a blade-paddle-type stirrer was used.

Suspensions of Comparative Examples 2 and 3 were prepared in the same manner as described above, except that only the type and amount of the polymeric suspending agent; the type and amount of the sweetener; the type and amount of the pH adjusting agent; and the amount of the flavor were varied. The specific composition of each experimental group is shown in Table 2 below.

The viscosity of each of the suspensions of Example 1 and Comparative Examples 2 and 3 was measured. Specifically, each of the suspension samples of Example 1 and Comparative Examples 1, 2, and 3 was left at 5 °C for 24 hours, and then the viscosity of each sample was measured using the viscometer RheolabQC with measuring cylinder CC27 under $25 \pm 0.1$ °C temperature conditions. Specific viscometer and conditions are the same as shown in Table 1 below.

[Table 1]

| Measuring Instrument | RheolabQC CC27 |
|---|---|
| Measuring temperature | 25 °C |
| Relaxation time | 24 hours |
| Parameters | 1) Thermal equilibrium time: 30 minutes<br>2) Pre-shearing at 100 s$^{-1}$, 30 seconds, and 5 measuring points<br>3) Shearing at 100 s$^{-1}$ to 1,400 s$^{-1}$, 60 seconds, and 20 measuring points |
| Viscosity value | The average of 20 measuring points |
| Units | mPa·s |

[Table 2]

| Composition | | Example 1 (w/w%) | Comparative Example 1 (w/w%) | Comparative Example 2 (w/w%) | Comparative Example 3 (w/w%) |
|---|---|---|---|---|---|
| API | | Montmorillonite 8.76% | Montmorilloni te 30% | Montmorilloni te 30% | Montmorilloni te 30% |
| Polymer Suspending agent | | Xanthan gum 0.22% | Maltitol liquid 25% | Xanthan gum 0.3% | Xanthan gum 0.25% |
| Low molecular weight suspending agent | | Glycerin 10.07% | - | - | - |
| Sweetener | | Sucrose 6.13% | Sodium saccharin 0.06% | Sucralose 0.0375% | Sucralose 0.0375% |
| Preservative | | Sodium benzoate 0.06% | Potassium sorbate 0.175% | Potassium sorbate 0.175% | Potassium sorbate 0.175% |
| Flavor | Vanilla flavor 0.53% | Aroma caramel 0.5% | Aroma caramel 1% | Aroma caramel 1% |
| | Strawberry essence 0.53% | | | |
| pH adjusting agent | | Sodium citrate 0.07% | Ascorbic acid 0.1% | Ascorbic acid 0.1% | Ascorbic acid 0.1% |
| pH adjusting agent | | Citric acid 0.11% | Citric acid 0.2 % | Citric acid 0.2% | Citric acid 0.2% |
| Carrier | | Purified water to 100% | Purified water to 100% | Purified water to 100% | Purified water to 100% |

(continued)

| Composition | Example 1 (w/w%) | Comparative Example 1 (w/w%) | Comparative Example 2 (w/w%) | Comparative Example 3 (w/w%) |
|---|---|---|---|---|
| Viscosity | 12.39-85.967 mPa·s | 650.84-3594.6 mPa·s | 2345.4 mPa·s | 2938.7 mPa·s |

As shown in Table 2 above, it was confirmed that the known smectite suspensions of Comparative Examples 1, 2, and 3 had a viscosity that is equal to or greater than 80 times to 300 times that of the smectite suspension of Example 1. The viscosity of each of the suspensions of Comparative Examples 1 to 3 is so high that it is difficult for a user to take the suspensions due to difficulty in swallowing the same, and stirring is not performed well in the preparation of the suspensions, resulting in an increased defect rate of products. Thus, it has been determined that it is difficult to use the suspensions of Comparative Examples 1 to 3 as pharmaceutical preparations in that the introduction of new equipment is required to prepare uniformly dispersed suspensions. However, it was confirmed that by containing both the polymeric suspending agent and the low molecular suspending agent, the smectite suspension of the present invention had a viscosity between 10 mPa·s and 90 mPa·s, and thus has formulation properties suitable for use as a pharmaceutical preparation, and it was examined whether the types and amounts of the suspending agents affected formulation properties thereof.

**[Experimental Example 2] Confirmation of Difference in Formulation Properties according to Types and Amounts of Suspending Agents**

Experimental Example 2-1

Suspensions are required to satisfy not only the viscosity but also a sinking rate of a certain level or higher for formulation stability. In particular, for the approval of medicines, the sinking rate is required to be 0.9 or more. Therefore, in order to prepare a suspension that satisfies these conditions, the suspension was prepared by adjusting the contents of the polymeric suspending agent and the low molecular suspending agent, and the formulation properties of each suspension were examined. In particular, each suspension was prepared using the same method as that used to prepare the suspension of Example 1, except that the amount of each composition was varied. The specific compositions of each experimental group are the same as shown in Table 3 below.

[Table 3]

| Composition | Ingredients | Example 1 | Example 2 | Example 3 | Comparative Example 4 | Comparative Example 5 | Comparative Example 6 | Comparative Example 7 |
|---|---|---|---|---|---|---|---|---|
| API | Montmoril lonite | 3.0 g | 3.0 g | 3.0 g | 3.0 g | 3.0 g | 3.0 g | 3.0 g |
| Polymer suspending agent | Xanthan gum | 0.075 g | 0.030g | 0.120 g | - | 0.015 g | 0.135 g | 0.150 g |
| Low molecular weight suspending agent | Glycerin | 3.45 g | 3.495 g | 3.405 g | 3.525 g | 3.510 g | 3.390 g | 3.375 g |
| Sweetener | Sucrose | 2.1 g | 2.1 g | 2.1 g | 2.1 g | 2.1 g | 2.1 g | 2.1 g |
| Preservative | Sodium benzoate | 0.0212 4 g | 0.0212 4 g | 0.0212 4 g | 0.0212 4 g | 0.0212 4 g | 0.0212 4 g | 0.0212 4 g |
| Flavor | Vanilla flavor | 0.18 g | 0.18 g | 0.18 g | 0.18 g | 0.18 g | 0.18 g | 0.18 g |
| Flavor | Strawberry essence | 0.18 g | 0.18 g | 0.18 g | 0.18 g | 0.18 g | 0.18 g | 0.18 g |
| Colorant | Erythrosine | 0.0013 5 g | 0.0013 5 g | 0.0013 5 g | 0.0013 5 g | 0.0013 5 g | 0.0013 5 g | 0.0013 5 g |
| pH adjusting agent | Citric acid | 0.0375 7 g | 0.0375 7 g | 0.0375 7 g | 0.0375 7 g | 0.0375 7 g | 0.0375 7 g | 0.0375 7 g |
| pH adjusting agent | Sodium citrate | 0.0224 7 g | 0.0224 7 g | 0.0224 7 g | 0.0224 7 g | 0.0224 7 g | 0.0224 7 g | 0.0224 7 g |
| Carrier | Purified water | To 30 mL | To 30 mL | To 30 mL | To 30 mL | To 30 mL | To 30 mL | To 30 mL |

The sinking rate of each suspension prepared according to the compositions shown in Table 3 above was measured using the following method.

A sample was put in a 50 ml mass cylinder at room temperature, mixed while shaken vigorously for 1 minute to mark the height of the content, and this was denoted as $H_0$, and after left for 3 hours, the final height of the content was denoted as H, and the sinking rate was calculated using Equation 1 below.

$$[Equation\ 1]$$

$$Sinking\ rate = H/H_0$$

In addition, the viscosity of each suspension was measured using the following method. Samples were allowed to stand at 5 °C for 24 hours, and then the thermal equilibrium time was maintained for 30 minutes and the viscosity of each experimental group was obtained using a RheolabQC viscometer by measuring 20 stabilized sections within 60 seconds at a rate of 100 $s^{-1}$ to 1,400 $s^{-1}$ and a temperature of 25 °C.

[Table 4]

| Experimental group | Phase | pH | Sinking rate | Viscosity (mPa·s) (Rate: 100$s^{-1}$ to 1,400 $s^{-1}$) | Content of ingredient |
|---|---|---|---|---|---|
| Example 1 | Pink suspension | 6.33 | 0.99 | 12.39-85.967 | 103.42 |
| Example 2 | Pink suspension | 6.26 | 0.97 | 7.554-23.814 | 101.34 |
| Example 3 | Pink suspension | 6.25 | 0.99 | 13.49-123.14 | 101.15 |
| Comparative Example 4 | Pink suspension | 4.74 | 0.54 | 2.283-4.1567 | 97.63 |
| Comparative Example 5 | Pink suspension | 5.91 | 0.38 | 5.16-12.045 | 99.30 |
| Comparative Example 6 | Pink suspension | 6.50 | 0.98 | 21.46-138.55 | 95.79 |
| Comparative Example 7 | Pink suspension | 6.28 | 0.99 | 15.78-156.42 | 95.87 |

As shown in Table 4 above, it was confirmed that the experimental group (Comparative Example 4) not comprising the polymeric suspending agent and the experimental group (Comparative Example 5) comprising a very low content of the polymeric suspending agent exhibited a very poor sending rate. It was determined that the compositions of Comparative Examples 4 and 5 were unsuitable as drugs for the reason that the reduction in sinking rate deteriorates the stability and uniformity of a formulation, due to layer separation during storage of the formulation.

It was also confirmed that the higher the content of the polymeric suspending agent in the composition (Comparative Examples 6 and 7), the higher the viscosity of the suspension. In particular, in the case of Comparative Examples 6 and 7, the proportion of defective products, in which the content of a component in the sample is below the standard, was high in the preparation process. This is related to the ease of preparation and efficiency of the suspension, and due to the characteristics of the formulation, stirring is not performed well in the preparation process, and thus each component was not able to be uniformly dispersed in the suspension. However, as in Examples 1 to 3, it was confirmed that the suspensions comprising both the polymeric suspending agent and the low molecular suspending agent had an appropriate viscosity to be suitable for use as pharmaceutical agents and exhibited excellent content uniformity and a high sinking rate in the preparation thereof.

Experimental Example 2-2

To confirm whether the sinking rate is satisfied even when the polymer and low molecular weight suspending agents other than xanthan gum and glycerin are comprised, suspensions were prepared and the sinking rate of each suspension was examined.

Specifically, an appropriate amount of purified water was put in a stainless steel container, xanthan gum was slowly added to the container according to the composition and stirred for 10 minutes, and then left for 24 hours to swell the resulting solution. After adding an appropriate amount of purified water to a liquid mixing tank, a sweetener was added to the liquid mixing tank according to the composition, stirred and dissolved. The swollen xanthan gum liquid was added to the liquid mixing tank, stirred, and homogenized, and then glycerin was added according to the composition, stirred, and homogenized. An appropriate amount of purified water was added to the liquid mixing tank, a pH adjusting agent was added to the liquid mixing tank according to the composition, stirred, and dissolved. The liquid mixing tank was heated to 90 °C for 45 minutes in the stirring state. An appropriate amount of purified water was put in a separate liquid mixing tank, and smectite was slowly added in aliquots according to the composition, was uniformly dispersed for 30 minutes while continuously stirring, and then the aqueous smectite dispersion was added to the liquid mixing tank comprising the suspension. Thereafter, the liquid mixing tank was heated to 90 °C for 45 minutes in the stirring state, and then cooled to 35 °C while continuously stirring. An appropriate amount of purified water was added to the liquid mixing tank, and a preservative and a colorant were added according to the composition and dissolved, and then the resulting solution was added to the liquid mixing tank and mixed therein. The stirring was stopped, purified water was added according to the composition and then further stirred for 15 minutes, which was then injected into a medicinal composite film to prepare a suspension preparation. Each suspension was prepared in accordance with the compositions shown in Table 5, but the compositions of Table 5 for the preparation of each suspension were converted into values with respect to 300 ml, thereby preparing 300 ml of suspensions.

The sinking rate of each of the prepared suspensions was measured using the same method as that used in Experimental Example 2-1.

[Table 5]

| Composition | Ingredients | Example 4 | Example 5 | Example 6 | Example 7 | Comparative Example 8 | Comparative Example 9 |
|---|---|---|---|---|---|---|---|
| API | Montmorillonite | 3.0 g | 3.0 g | 3.0 g | 3.0 g | 3.0 g | 3.0 g |
| Polymer suspending agent | | RC591 0.6 g | Xanthan gum 0.1 g | HPC 1.2 g | Xanthan gum 0.05 q | Xanthan gum 0.05 q | - |
| Low molecular weight suspending agent | | Sorbitol 3.0 g | Glycerin 3.45 g | Glycerin 2.0 g | Glycerin 4.5 g | - | Glycerin 6.0 g |
| Sweetener | Sucrose | 2.1 | 2.1 | 2.1 | 2.1 | 2.1 g | 2.1 g |
| Preservative | Sodium benzoate | 0.02124 g | 0.02124 g | 0.02124 g | 0.02124 g | 0.02124 g | 0.02124 g |
| Flavor | Vanilla flavor | 0.18 g | 0.18 g | 0.18 g | 0.18 g | 0.18 g | 0.18 g |
| Flavor | Strawberry essence | 0.18 g | 0.18 g | 0.18 g | 0.18 g | 0.18 g | 0.18 g |
| Colorant | Erythrosine | 0.00135 g | 0.00135 g | 0.00135 g | 0.00135 g | 0.00135 g | 0.00135 g |
| pH adjusting agent | Citric acid | 0.03757 g | 0.03757 g | 0.03757 g | 0.03757 g | 0.03757 g | 0.03757 g |
| pH adjusting agent | Sodium citrate | 0.02247 g | 0.02247 g | 0.02247 g | 0.02247 g | 0.02247 g | 0.02247 g |
| Carrier | Purified water | To 30 mL | To 30 mL | To 30 mL | To 30 mL | To 30 mL | To 30 mL |
| -Contents of ingredients per 1 sachet (30 mL, 34.26 g) of finished product<br>-The specific gravity of each suspension composition was 1.142±0.030 g/mL | | | | | | | |
| Sinking rate | | 0.96 | 0.98 | 0.98 | 0.93 | 0.85 | 0.30 |

As shown in Table 5, it was confirmed that as in Comparative Examples 8 and 9, the suspension comprising the polymeric suspending agent or the low molecular suspending agent alone had a low sinking rate, i.e., less than 0.9, and thus was not suitable for use as a medicine, but all the compositions of Examples 4 to 7 according to the present invention which comprised both the polymeric suspending agent and the low molecular suspending agent exhibited very high sinking rates.

**[Experimental Example 3] Confirmation of Difference in Formulation Properties according to Content of Low molecular weight Suspending Agent**

Based on the above experiments, experiments were conducted on the composition comprising both the polymer and low molecular weight suspending agents in order to confirm the effect of the content of the low molecular suspending agent on formulation properties of the composition.

Specifically, each suspension was prepared according to the composition shown in Table 6 below using the same method as that used in Example 1 of Experimental Example 1, except that only the content of the low molecular suspending agent was varied (other ingredients except for the polymer and low molecular weight suspending agents were the same as shown in Table 5).

[Table 6]

| Compositio n | Ingredien ts | Comparativ e Example 10 | Comparativ e Example 11 | Example 1 | Comparative Example 12 | Comparative Example 13 |
|---|---|---|---|---|---|---|
| Polymer suspending agent | Xanthan gum | 0.075 g | 0.075 g | 0.075 g | 0.075 g | 0.075 g |
| Low molecular weight suspending agent | Glycerin | 1.45 g | 2.45 g | 3.450 g | 4.45 g | 5.45 g |
| -Contents of ingredients per 1 sachet (30 mL, 33.75 g) of finished product -The specific gravity of each suspension composition was 1.125±0.030 g/mL | | | | | | |

The results of measuring the texture, aroma, and overall preference of each of the prepared suspensions are shown in Table 7 below. The panel consisted of a total of 10 male and female adults, the scores of the panel for each experimental group based on 10 points were added up and divided by 10, thereby obtaining an average.

[Table 7]

| Experimental groups | Texture | Aroma | Overall preference |
|---|---|---|---|
| Comparative Example 10 | 6.6 | 9.8 | 16.4 |
| Comparative Example 11 | 7.4 | 9.5 | 16.9 |
| Example 1 | 9.7 | 9.7 | 19.4 |
| Comparative Example 12 | 6.3 | 9.2 | 15.5 |
| Comparative Example 13 | 4.8 | 8.8 | 13.6 |

As shown in Table 7 above, as the content of glycerin increased to a certain level or higher, texture rapidly worsened, but the suspension of Example 1 exhibited significantly enhanced texture and high overall preference compared to the suspensions of Comparative Examples 10 to 14.

**Claims**

1. A smectite suspension comprising smectite; and a polymeric suspending agent and a low molecular suspending agent,
   wherein

   the total amount of the polymeric suspending agent and the low molecular suspending agent is in the range from 10 %(w/v) to 15 %(w/v) with respect to the total suspension composition,
   the low molecular suspending agent is glycerin and/or sorbitol,
   the low molecular suspending agent is comprised in an amount of 8.5 %(w/v) to 14 %(w/v) with respect to the total amount of the smectite suspension, and
   the polymeric suspending agent is comprised in an amount of greater than 0.05 %(w/v) to less than 0.45 %(w/v) with respect to the total amount of the suspension.

2. The smectite suspension according to claim 1, wherein the smectite is comprised in an amount of 8 %(w/v) to 15 % (w/v) with respect to a total amount of the suspension.

3. The smectite suspension according to any one of claims 1 to 2, wherein the polymeric suspending agent is one or more selected from the group consisting of microcrystalline cellulose, carboxymethylcellulose, hydroxypropyl cellulose, xanthan gum, carrageenan, and carbomer.

4. The smectite suspension according to any one of claims 1 to 3, wherein the suspension further comprises one or more ingredients selected from the group consisting of a sweetener, a preservative, a flavor, a pH adjusting agent, and a colorant.

5. The smectite suspension according to any one of claims 1 to 4, wherein the polymeric suspending agent is xanthan gum, and the low molecular suspending agent is glycerin.

6. The smectite suspension according to claim 5, wherein the suspension comprises 8 %(w/v) to 12 %(w/v) of smectite, 0.1 %(w/v) to 0.4 %(w/v) of xanthan gum as the polymeric suspending agent, and 8.5 %(w/v) to 14 %(w/v) of glycerin as the low molecular suspending agent, with respect to the total amount of the suspension.

7. The smectite suspension according to claim 6, wherein the suspension further comprises 5 % (w/v) to 9 %(w/v) of sucrose, 0.05 %(w/v) to 0.09 %(w/v) of sodium benzoate, 0.09 %(w/v) to 0.35 %(w/v) of citric acid, and 0.055 %(w/v) to 0.095 %(w/v) of sodium citrate.

8. A method of preparing a smectite suspension, the method comprising:

preparing a mixed suspending agent by mixing a swollen polymer suspending agent and a low molecular suspending agent and heating the resulting mixture at 90 °C to 95 °C for 25 minutes to 45 minutes; and
preparing a smectite suspension by mixing the mixed suspending agent with an aqueous smectite dispersion and heating the resulting mixture at 85 °C to 95 °C for 15 minutes to 45 minutes,
wherein
the total amount of the polymeric suspending agent and the low molecular suspending agent is in the range from 10 %(w/v) to 15 %(w/v) with respect to the total suspension composition,
the low molecular suspending agent is glycerin and/or sorbitol,
the low molecular suspending agent is comprised in an amount of 8.5 %(w/v) to 14 %(w/v) with respect to the total amount of the smectite suspension, and
the polymeric suspending agent is comprised in an amount of greater than 0.05 % (w/v) to less than 0.45 %(w/v) with respect to the total amount of the suspension.

**Patentansprüche**

1. Smektitsuspension, umfassend Smektit, und ein polymeres Suspensionsmittel und ein niedermolekulares Suspensionsmittel,
wobei

die Gesamtmenge des polymeren Suspensionsmittels und des niedermolekularen Suspensionsmittels im Bereich von 10 % (w/v) bis 15 % (w/v) in Bezug auf die gesamte Suspensionszusammensetzung liegt,
das niedermolekulare Suspensionsmittel Glycerin und/oder Sorbit ist,
das niedermolekulare Suspensionsmittel in einer Menge von 8,5 % (w/v) bis 14 % (w/v) in Bezug auf die Gesamtmenge der Smektitsuspension enthalten ist, und
das polymere Suspensionsmittel in einer Menge von mehr als 0,05 % (w/v) bis weniger als 0,45 % (w/v) in Bezug auf die Gesamtmenge der Suspension enthalten ist.

2. Smektitsuspension nach Anspruch 1, wobei das Smektit in einer Menge von 8 % (w/v) bis 15 % (w/v) in Bezug auf die Gesamtmenge der Suspension enthalten ist.

3. Smektitsuspension nach einem der Ansprüche 1 bis 2, wobei das polymere Suspensionsmittel eines oder mehrere, ausgewählt aus der Gruppe bestehend aus mikrokristalliner Cellulose, Carboxymethylcellulose, Hydroxypropylcellulose, Xanthangummi, Carrageen und Carbomer, ist.

**4.** Smektitsuspension nach einem der Ansprüche 1 bis 3, wobei die Suspension weiter einen oder mehrere Inhaltsstoffe umfasst, ausgewählt aus der Gruppe bestehend aus einem Süßungsmittel, einem Konservierungsmittel, einem Aromastoff, einem pH-Einstellmittel und einem Farbstoff.

**5.** Smektitsuspension nach einem der Ansprüche 1 bis 4, wobei das polymere Suspensionsmittel Xanthangummi ist und das niedermolekulare Suspensionsmittel Glycerin ist.

**6.** Smektitsuspension nach Anspruch 5, wobei die Suspension 8 % (w/v) bis 12 % (w/v) Smektit, 0,1 % (w/v) bis 0,4 % (w/v) Xanthangummi als polymeres Suspensionsmittel und 8,5 % (w/v) bis 14 % (w/v) Glycerin als niedermolekulares Suspensionsmittel, in Bezug auf die Gesamtmenge der Suspension, umfasst.

**7.** Smektitsuspension nach Anspruch 6, wobei die Suspension weiter 5 % (w/v) bis 9 % (w/v) Saccharose, 0,05 % (w/v) bis 0,09 % (w/v) Natriumbenzoat, 0,09 % (w/v) bis 0,35 % (w/v) Zitronensäure und 0,055 % (w/v) bis 0,095 % (w/v) Natriumcitrat umfasst.

**8.** Verfahren zur Herstellung einer Smektitsuspension, wobei das Verfahren Folgendes umfasst:

Herstellen eines gemischten Suspensionsmittels durch Mischen eines gequollenen polymeren Suspensionsmittels und eines niedermolekularen Suspensionsmittels und Erhitzen der resultierenden Mischung auf 90 °C bis 95 °C für 25 Minuten bis 45 Minuten; und
Herstellen einer Smektitsuspension durch Mischen des gemischten Suspensionsmittels mit einer wässrigen Smektitdispersion und Erhitzen der resultierenden Mischung bei 85 °C bis 95 °C für 15 Minuten bis 45 Minuten, wobei
die Gesamtmenge des polymeren Suspensionsmittels und des niedermolekularen Suspensionsmittels im Bereich von 10 % (w/v) bis 15 % (w/v) in Bezug auf die gesamte Suspensionszusammensetzung liegt,
das niedermolekulare Suspensionsmittel Glycerin und/oder Sorbit ist,
das niedermolekulare Suspensionsmittel in einer Menge von 8,5 % (w/v) bis 14 % (w/v) in Bezug auf die Gesamtmenge der Smektitsuspension enthalten ist, und
das polymere Suspensionsmittel in einer Menge von mehr als 0,05 % (w/v) bis weniger als 0,45 % (w/v) in Bezug auf die Gesamtmenge der Smektitsuspension enthalten ist.

**Revendications**

**1.** Suspension de smectite comprenant de la smectite ; et un agent polymérique de suspension et un agent de suspension de faible poids moléculaire,
dans laquelle

la quantité totale de l'agent polymérique de suspension et de l'agent de suspension de faible poids moléculaire est dans la plage de 10 % (p/v) à 15 % (p/v) par rapport à la composition totale de la suspension,
l'agent de suspension de faible poids moléculaire est de la glycérine et/ou du sorbitol,
l'agent de suspension de faible poids moléculaire est compris en une quantité de 8,5 % (p/v) à 14 % (p/v) par rapport à la quantité totale de la suspension de smectite, et
l'agent polymérique de suspension est compris en une quantité supérieure à 0,05 % (p/v) à inférieure à 0,45 % (p/v) par rapport à la quantité totale de la suspension.

**2.** Suspension de smectite selon la revendication 1, dans laquelle la smectite est comprise en une quantité de 8 % (p/v) à 15 % (p/v) par rapport à une quantité totale de la suspension.

**3.** Suspension de smectite selon l'une quelconque des revendications 1 à 2, dans laquelle l'agent polymérique de suspension est un ou plusieurs sélectionnés dans le groupe consistant en la cellulose microcristalline, la carboxyméthylcellulose, l'hydroxypropylcellulose, la gomme de xanthane, le carraghénane et le carbomère.

**4.** Suspension de smectite selon l'une quelconque des revendications 1 à 3, dans laquelle la suspension comprend en outre un ou plusieurs ingrédients sélectionnés dans le groupe consistant en un édulcorant, un conservateur, un arôme, un agent d'ajustement de pH et un colorant.

**5.** Suspension de smectite selon l'une quelconque des revendications 1 à 4, dans laquelle l'agent polymérique de suspension est de la gomme de xanthane, et l'agent de suspension de faible poids moléculaire est de la glycérine.

6. Suspension de smectite selon la revendication 5, dans laquelle la suspension comprend 8 % (p/v) à 12 % (p/v) de smectite, 0,1 % (p/v) à 0,4 % (p/v) de gomme de xanthane en tant qu'agent polymérique de suspension et 8,5 % (p/v) à 14 % (p/v) de glycérine en tant qu'agent de suspension de faible poids moléculaire, par rapport à la quantité totale de la suspension.

7. Suspension de smectite selon la revendication 6, dans laquelle la suspension comprend en outre 5 % (p/v) à 9 % (p/v) de sucrose, 0,05 % (p/v) à 0,09 % (p/v) de benzoate de sodium, 0,09 % (p/v) à 0,35 % (p/v) d'acide citrique et 0,055 % (p/v) à 0,095 % (p/v) de citrate de sodium.

8. Procédé de préparation d'une suspension de smectite, le procédé comprenant :

la préparation d'un agent de suspension mélangé par mélange d'un agent polymérique de suspension gonflé et d'un agent de suspension de faible poids moléculaire et chauffage du mélange résultant à 90 °C à 95 °C pendant 25 minutes à 45 minutes ; et
la préparation d'une suspension de smectite par mélange de l'agent de suspension mélangé avec une dispersion aqueuse de smectite et chauffage du mélange résultant à 85 °C à 95 °C pendant 15 minutes à 45 minutes, dans laquelle
la quantité totale de l'agent polymérique de suspension et de l'agent de suspension de faible poids moléculaire est dans la plage de 10 % (p/v) à 15 % (p/v) par rapport à la composition totale de la suspension,
l'agent de suspension de faible poids moléculaire est de la glycérine et/ou du sorbitol,
l'agent de suspension de faible poids moléculaire est compris en une quantité de 8,5 % (p/v) à 14 % (p/v) par rapport à la quantité totale de la suspension de smectite, et
l'agent polymérique de suspension est compris en une quantité supérieure à 0,05 % (p/v) à inférieure à 0,45 % (p/v) par rapport à la quantité totale de la suspension.

【Fig. 01】

【Fig. 02】

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 2386289 A1
- CN 102218078 A
- WO 2005041876 A3
- WO 2010102923 A3
- CN 106281044 A
- US 4942042 A
- CN 106265724 A
- CN 101129326 A
- CN 101065111 A
- US 2015306113 A1
- US 7135197 B2
- US 5272137 A

**Non-patent literature cited in the description**

- Fenaroli's Handbook of Flavor Ingredients. CRC Pres, 1975